# EUROPÄISCHE PATENTSCHRIFT

(11) **EP 3 571 262 B1**
(45) Veröffentlichungstag und Bekanntmachung des Hinweises auf die Patenterteilung: **21.12.2022**
(21) Anmeldenummer: 18701312.3
(22) Anmeldetag: 19.01.2018
(51) Int. Cl.: C09K 11/02, C09K 11/08, G01N 33/58

(54) **VERWENDUNG EINES ZWITTERIONISCHE NANOPARTIKEL**
USE OF ZWITTERIONIC NANOPARTICLES
UTILIZATION DES NANOPARTICULES ZWITTÉRIONIQUES

(30) Priorität: 20.01.2017 DE 102017101057
(43) Veröffentlichungstag der Anmeldung: 27.11.2019
(73) Patentinhaber: Fraunhofer-Gesellschaft zur Förderung der angewandten Forschung e.V., 80686 München (DE)
(72) Erfinder: SCHOTTEN, Theo, 20146 Hamburg (DE); STEUTER, Michaela, 20146 Hamburg (DE); MERKL, Jan-Philip, 20146 Hamburg (DE); WELLER, Horst, 20146 Hamburg (DE)
(74) Vertreter: DTS Patent- und Rechtsanwälte Schnekenbühl und Partner mbB
(86) Internationale Anmeldenummer: PCT/EP2018/051284
(87) Internationale Veröffentlichungsnummer: WO 2018/134342

(56) Entgegenhaltungen:
- WO-A2-2013/090601
- US-A1- 2010 258 789
- US-A1- 2010 316 797
- US-A1- 2012 052 513
- ELEONORA MURO ET AL: "Small and Stable Sulfobetaine Zwitterionic Quantum Dots for Functional Live-Cell Imaging", JOURNAL OF THE AMERICAN CHEMICAL SOCIETY, Bd. 132, Nr. 13, 7. April 2010 (2010-04-07), Seiten 4556-4557, XP055000735, ISSN: 0002-7863, DOI: 10.1021/ja1005493

## Beschreibung

### TECHNISCHES FELD

Die vorliegende Erfindung betrifft zwitterionische Nanopartikel. Insbesondere werden zwitterionische Verbindungen beschrieben, die die Oberfläche von Nanopartikeln bedecken. Weiterhin beschreibt die vorliegende Erfindung den Gebrauch einer solchen Zubereitung sowie Wege zu ihrer Herstellung.

### TECHNISCHER HINTERGRUND

Nanopartikel sind von großem Interesse, da sie breite technische Anwendung versprechen, wie in Bildwiedergabegeräten, als Informationsspeicher, als biologische Markierungsmittel, in der diagnostischen Bildgebung, als Wirkstofftransporter, als Theranostika, in der Photovoltaik, als Sensoren und als Katalysatoren. Nanopartikel können infolge ihrer kleinen Abmessungen physikochemische Eigenschaften aufweisen, die zwischen denen von Molekülen und makroskopischen Materialien liegen. Zum Beispiel können Nanopartikel aus Halbleitermaterialien Quanteneffekte, sowohl des Elektrons als und somit auch der Elektronenlücke in allen drei Dimensionen aufweisen, die zu einem Anstieg der Bandlücke des Materials bei sinkender Größe des Kristalliten führt. Auch die magnetischen Eigenschaften der Materie ändern sich dramatisch in Abhängigkeit von der Partikelgröße. Ebenso hängt die Plasmonabsorption von Nanopartikeln, z. B. Goldnanopartikeln überwiegend von der Größe und Form besagter Partikel ab.

Die biologischen Effekte von Nanopartikeln, wie das Durchdringungsvermögen von biologischen Schranken, wie Membranen oder die Haut oder die Toxizität von Nanopartikeln hängen jedoch nicht nur von den Partikeleigenschaften an sich, sondern auch von Veränderungen ab, die durch das Einbringen besagter Partikel in biologische Systeme eintreten. Insbesondere können spontane Auflagerungen biologischer Moleküle stattfinden, die peptidische, protein-, nukleinsäure-, oder kohlenhydrat-artige Strukturelemente aufweisen, wodurch sich eine "biomolekulare Korona" um das ursprüngliche Nanopartikel ausbilden kann, die diesem neuartige, unvorhersehbare und daher meist unerwünschte Eigenschaften verleiht und das weitere biologische Verhalten besagter Partikel maßgeblich bestimmt. Dieser Prozess ist dem Fachmann als Opsonisierung, die sich daraus ergebenden, unerwünschten Eigenschaften u.a. als "Fouling" bekannt. Besagte unerwünschte Effekte stellen ein großes Hindernis bei der Nutzbarmachung von Nanopartikeln für bio-medizinische Anwendungen dar, da für die gezielte Beeinflussung biologischer Vorgänge eine hohe Selektivität bzw. Spezifität besagter Nanopartikel für die biologischen Zielstrukturen, wie Zellen, Gewebe, Organe, Tumoren usw. unabdingbar ist.

Daher wurden zahlreiche Versuche unternommen, besagte unerwünschte Prozesse durch eine entsprechende Oberflächenausstattung besagter Nanopartikel zu unterdrücken und so die damit verbundenen Nachteile zu beseitigen. Insbesondere wurden Poly(ethylenglycol) (PEG) und Oligo(ethylenglycol) (OEG) zur Abdeckung von Nanopartikel-Oberflächen vorgeschlagen. Ebenso wurden zwitterionische Strukturen für diesen Zweck beschrieben, wonach letztere den Stand der Technik abbilden. So beansprucht WO2013090601 (A2) Nanopartikel mit zwitterionischen Liganden, insbesondere um einen geringen Grad von unspezifischer Proteinabsorption zu erreichen. Weiterhin beansprucht WO2013090601 (A2) die Funktionalisierung besagter Nanopartikel mit einer oder mehreren funktionellen Gruppen, um besagten Nanopartikeln die gewünschten Eigenschaften, wie die Bindung an ein Zielmolekül oder an einen Rezeptor zu verleihen.

MURO, E. et al. Kleine und stabile sulfobetain zwitterionische Quantenpunkte für funktionelle lebendzell-Bildgebung. Journal of the American Chemical Society. April 2010, Band 132, Nr 13, Seiten 4556 - 4557

US2012/052513 A1 beschreibt Gold Sub-Nanokluster und deren Anwendung.

US2010/316797 A1 beschreibt die Bildung von Glutathion-gekappten und metalldotierten Zinkselenid/Zinksulfid Kern-Schalen Quantenpunkten in wässriger Lösung.

WO2013090601 A2 beschreibt kompakte Nanopartikel für biologische Applikationen.

Somit hat die Belegung von Nanopartikeln mit zwitterionischen Strukturen zum Ziel, die Oberfläche besagter Nanopartikel gegenüber biologischen Einflüssen zu passivieren bzw. zu inertisieren. Dieser Effekt wird in der angelsächsischen Literatur mit "Stealth" bezeichnet. Der gewünschte Effekt ist, dass sich solcher Art inertisierte Nanopartikel zunächst indifferent gegenüber biologischen Einflüssen verhalten, somit für das jeweilige biologische System "unsichtbar" sind und erst durch Anbindung spezifischer Erkennungsmotive für den jeweiligen Einsatzzweck ertüchtigt werden.

Die US 2014/0235803 A1 betrifft nanopartikelbasierte biotechnologische Anwendungen mit gegenüber Opsonisierung unempfindlichen Materialien ("non-fouling materials"), wie Poly(ethylene glycol) (PEG) und oligo(ethylene glycol) (OEG).

Es ist daher die Aufgabe der vorliegenden Erfindung zwitterionische Nanopartikel der eingangs genannten Art in vorteilhafter Weise weiterzubilden, insbesondere dahingehend, dass eine verbesserte und alternative Anwendungsmöglichkeit von Nanopartikeln ermöglicht wird.

### ZUSAMMENFASSUNG DER VORLIEGENDEN ERFINDUNG

Diese Aufgabe wird erfindungsgemäß durch die Verwendung eines zwitterionischen Nanopartikels mit den Merkmalen des Anspruchs 1 gelöst. Danach ist die Verwendung eines zwitterionischen Nanopartikels zur unspezifischen Aufnahme in wenigstens eine Zelle vorgesehen, wobei das zwitterionische Nanopartikel wenigstens einen Nanopartikel und eine betainische Hülle aufweist, die den Nanopartikel umschließt, wobei das Nanopartikel ein lumineszenter Halbleiter Nanokristall (SCNC) ist.

Weitere Ausgestaltungen der vorliegenden Erfindung sind Gegenstand der abhängigen Ansprüche.

Ein Verfahren zur Anbindung eines zwitterionischen Nanopartikels oder einer Zusammensetzung an ein biologisches Molekül oder eine Vielzahl biologischer Moleküle ist möglich.

Die Verwendung eines zwitterionischen Nanopartikels sowie einer Zusammensetzung für ein biologisches Ereignis oder eine biologische Anwendung ist möglich.

Überraschend wurde gefunden, dass die vom Stand der Technik beschriebene Vorgehensweise in der vorliegenden Erfindung nicht zu den erwarteten Effekten führte, sondern dass besagte, mit zwitterionischen Strukturen belegte Nanopartikel vielmehr auch ohne Modifikation mit spezifischen Erkennungsmotiven eine überraschend ausgeprägte, strukturabhängige Selektivität bezüglich der Aufnahme in Zellen aufwiesen. Somit eignet sich die vorliegende Erfindung hervorragend dazu, durch Strukturvariationen der zwitterionischen Motive auf der Oberfläche von Nanopartikeln, die Aufnahme besagter Nanopartikel in Zellen gezielt zu steuern. Eine solche Anwendung wurde bisher nicht beschrieben und ist somit neu.

### KURZE BESCHREIBUNG DER ABBILDUNGEN

In den Figuren zeigen:
- Fig. 1: eine Quantifizierung der Toxizitätstests der QDQRs-17a und QDQRs-17b sowie Cadmiumchlorid;
- Fig. 2: mikroskopische Aufnahmen der A549-Zellen nach 16-stündiger Inkubationszeit mit unterschiedlichen Konzentrationen von QDQRs-17a;
- Fig. 3: mikroskopische Aufnahmen der A549-Zellen nach 16-stündiger Inkubationszeit mit unterschiedlichen Konzentrationen Cadmiumchlorid;
- Fig. 4: zeigt konfokalmikroskopische Aufnahmen von A549-Zellen nach der Inkubation; und
- Fig. 5: zeigt konfokalmikroskopische Aufnahmen von A549-Zellen nach der Inkubation mit 100 nM QDQRs-17b.

### AUSFÜHRLICHE BESCHREIBUNG DER IN DER VORLIEGENDEN ERFINDUNG VERWENDETEN BEGRIFFE

Die vorliegende Erfindung beschreibt Nanopartikel mit einer organischen zwitterionischen Hülle, wobei besagte Nanopartikel sich für den Transport von Molekülen, insbesondere Wirkstoffmolekülen eignen und optional superparamagnetische, plasmonische, oder fluoreszente Eigenschaften aufweisen können, einschließlich Kombinationen besagter Eigenschaften. Nanopartikel im Sinne der Erfindung sind räumliche Objekte, die eine Ausdehnung von 1 bis 1000 nm in wenigstens einer Raumrichtung haben. Besagte Nanopartikel können sowohl als feste Partikel, als auch als Hohlkörper in verschiedenen Formen vorliegen, die sphärische, längliche, z. B. stäbchenförmige, elliptische, eiförmige, hantelförmige, ringförmige, würfelförmige oder würfelähnliche, prismatische oder zylindrische, regelmäßig oder unregelmäßig mehrfach facettierte geometrische Körper, vieleckige Platten, von z. B. dreieckiger, quadratischer oder sechseckiger Form, Blättchen, vielarmige oder sternförmige Körper, wie z. B. Tetrapoden, oder drahtartige Gestalt usw. aufweisen. Besagte Nanopartikel können optional mit mindestens einer Schale umhüllt sein, die aus einem anderen Material als der Kern besteht. Die Bezeichnung "Nanopartikel" schließt die Begriffe "Nanokristall" oder "ultrakleines Partikel" mit ein.

Der detaillierten Beschreibung der Erfindung vorangestellt sei, dass diese Erfindung nicht auf die Methoden, Geräte oder Zubereitungen beschränkt ist, da diese selbstverständlich veränderbar sind. Weiterhin sollen die zur Beschreibung der Erfindung verwendeten Begriffe lediglich dazu dienen, bestimmte Ausführungsformen zu erläutern und den Geltungsbereich der Erfindung keinesfalls einschränken. So schließt der bestimmte oder unbestimmte Artikel im Singular "ein" oder "eine" und "der", "die", "das" den Plural ebenfalls mit ein, sofern der Zusammenhang das Gegenteil nicht klar erfordert. Demzufolge schließt z. B. der Bezug "eine Zelle" eine Vielzahl von Zellen, "ein Nanopartikel" eine Vielzahl von Nanopartikeln, "ein Biomolekül", wie beispielsweise DNS, Protein, Peptid, Kohlenhydrat usw. eine Vielzahl von Biomolekülen mit ein, usw.

Alle technischen und wissenschaftlichen Begriffe haben jene Bedeutung, so wie der Fachmann des Sachgebiets der vorliegenden Erfindung sie versteht, sofern nicht anders definiert oder der Zusammenhang eine andere Bedeutung nahelegt. Obwohl auch jede andere Methode oder andere Stoffe, ähnlich oder gleichwertig den hier beschriebenen, geeignet sind die Erfindung auszuüben oder zu erproben, werden im folgenden die bevorzugten Methoden und Stoffe beschrieben.

Alle Publikationen, die hier genannt werden, sind hiermit durch Referenzierung einbezogen für den Zweck der Offenbarung und der Beschreibung der einzelnen Materialien und Verfahrensweisen, für welche die entsprechende Referenz auch zitiert wurde.

Diese Publikationen, wie sie hier beschrieben werden, sind lediglich bereitgestellt für deren Offenbarung vor dem Anmeldetag der vorliegenden Erfindung. Hieraus kann jedoch nicht hergeleitet werden, was als Eingeständnis dahingehend gewertet werden kann, dass die Erfindung nicht berechtigt ist, eine derartige Offenbarung als Stand der Technik vorzudatieren. Beim Beschreiben der vorliegenden Erfindung werden die nachstehenden Begriffe benutzt und sollen wie nachfolgend definiert verstanden werden.

Der Begriff "anorganischer Nanokristall" bedeutet ein Partikel, allgemein ein Halbleiter-Partikel, oder ein nanokristalliner Partikel, dem keine Halbleitermaterialien zugrunde liegen, insbesondere ein seltenerd-dotierter Metalloxid-Partikel, oder ein seltenerd-dotierter, salzartiger Partikel, oder ein oxidischer, oder metallischer Partikel mit einem Durchmesser im Bereich von 1 nm bis 1000 nm, vorzugsweise im Bereich von ungefähr 2 nm bis 100 nm. Die Begriffe "Halbleiter Nanokristall" und "Quantum Dot (Quanten-Punkt)", "Quantum Rod, (Quanten-Stäbchen)", "Quantum Dot Rod, (Quantenpunkt/stäbchen)" oder die Abkürzungen QD, QDs, QR, QRs, QDR, QDRs, SCNC oder SCNCs werden hierbei untereinander austauschbar benutzt und bezeichnen Halbleiternanopartikel, die aus einem anorganischen Stoff bestehen, der luminiszent ist (d. h. der nach Anregung in der Lage ist, elektromagnetische Strahlung auszusenden), und umfasst einen inneren Kern aus einem oder mehreren ersten Halbleitermaterialien, der wahlweise mit einer Umhüllung oder "Schale" eines zweiten Halbleitermaterials umschlossen ist. Beispielsweise kann das umhüllende Schalenmaterial eine Bandlücken-Energie aufweisen, die höher ist, als jene des Kernmaterials und so ausgewählt ist, dass die Atomabstände ähnlich wie jene des Kernmaterials sind. Ein Halbleiternanokristall der von einer Halbleiterschale umhüllt ist wird als "Kern/Schale-Halbleiternanokristall bezeichnet.

Halbleiter werden insbesondere über ihre elektrische Leitfähigkeit beschrieben, die zwischen der eines Leiters und eines Nicht-Leiters liegt. Geeignete Halbleitermaterialien können folgende Materialien sein, ohne auf diese beschränkt zu sein, und bestehen aus einem ersten Element ausgewählt aus der Gruppe 2 und 12 des Periodensystems der Elemente und einem zweiten Element, ausgewählt aus der Gruppe 16 des Periodensystems (z. B. ZnS, ZnSe, ZnTe, CdS, CdSe, CdTe, HgS, HgSe, HgTe, MgS, MgSe, MgTe, CaS, CaSe, CaTe, SrS, SrSe, SrTe, BaS, BaSe, BaTe, und ähnliche); weiterhin Materialien bestehend aus einem ersten Element, ausgewählt aus der Gruppe 15 des Periodensystems der Elemente und einem zweiten Element aus der Gruppe 15 (z. B. GaN, GaP, GaAs, GaSb, InN, InP, InAs, InSb, usw.); weiterhin Materialien, bestehend aus der Gruppe 14 des Periodensystems der Elemente (Ge, Si usw.); Materialien wie z. B. PbS, PbSe, PbTe usw., Materialien wie CulnS, CuInGaS usw., sowie Legierungen und Mischungen davon. So wie hier verwendet, beziehen sich alle Angaben zum Periodischen System der Elemente und seinen Gruppen auf das neue IUPAC-Nummerierungssystem der Elementgruppen, wie in Handbook of Chemistry and Physics, 61 st 20 Edition (CRC Press, 2000) beschrieben.

Ein SCNC (semi conductor nano crystal) ist wahlweise von einer Schicht eines organischen Materials umschlossen. Diese organische Schicht kann aus einer Vielzahl von Stoffen bestehen und ist dadurch ausgezeichnet, dass sie eine Affinität zu der Oberfläche des SCNC aufweist. Allgemein kann das Beschichtungsmaterial aus kleinen organischen Einzelmolekül, Polymeren (oder Monomeren, die in einer Polymerisationsreaktion eingesetzt werden können), anorganischen Komplexen oder einer ausgedehnten kristallinen Struktur bestehen. Diese Beschichtung kann dazu dienen, die Löslichkeit eines derart beschichteten SCNC in einem gewählten Lösungsmittel, bestimmte Funktionalitäten oder Bindungseigenschaften zu vermitteln. Darüber hinaus kann die Beschichtung auch dazu dienen, die optischen Eigenschaften der SCNC einzustellen. Dementsprechend schließen die Begriffe "Halbleiternanokristall", "SCNC", "Quantum Dot (Quantenpunkt)", wie hier beschrieben, auch organisch ummantelte SCNC-Kerne, sowie organisch ummantelte Kern/Schale-SCNC mit ein.

"Monodisperse Partikel" schließen einen Bestand von Partikeln ein, in dem mindestens 60% vorzugsweise jedoch zwischen 75% und 90% der Partikel des Bestandes, einem gegebenen Größenbereich entsprechen. Ein Bestand monodisperser Partikel hat eine Abweichung des Partikeldurchmessers von weniger als 10%, vorzugsweise jedoch eine Abweichung von weniger als 5% vom quadratischen Mittelwert (rootmean-square, RMS) des Bestandes. Der Ausdruck "eine oder mehrere Größen von SCNC wird gleichbedeutend mit "ein oder mehrere Partikelgrößenverteilungen" verwendet. Der Fachmann wird verstehen, dass bestimmte Größen von Nanopartikeln tatsächlich als Partikelgrößenverteilungen erhalten werden.

Unter "Lumineszenz" wird ein Prozess verstanden, bei dem ein Gegenstand elektromagnetische Strahlung (Licht) aussendet. Lumineszenz entsteht, wenn ein System von einem angeregten Zustand in einen Zustand niedrigerer Energie übergeht, wobei die dabei abgegebene Energie in Form von Photonen freigesetzt wird. Diese Energiezustände können wahlweise in verschiedener Form vorliegen, als Schwingungsenergie oder Rotationsenergie oder elektronischer Natur sein, oder in jeglichen Kombination dieser Energien. Der Übergang, der die Lumineszenz verursacht, kann durch im System gespeicherte chemische Energie oder durch eine äußere Quelle ausgelöst werden. Die äußere Energiequelle kann in verschiedenen Formen, einschließlich chemischer, thermischer, elektrischer, magnetischer, elektromagnetischer, physikalischer, oder jeder anderen Form, die geeignet ist eine Anregung in einen höheren Energiezustand als den Grundzustand zu bewirken, zugeführt werden. Beispielsweise kann ein System durch die Aufnahme wenigstens eines Photons, durch Einbringen in ein elektrisches Feld, oder durch eine chemische Redox-Reaktion angeregt werden. Die Energie des während der Lumineszenz abgestrahlten Photons kann im Bereich niederenergetischer Mikrowellenstrahlung bis hochenergetischer Roentgenstrahlung liegen. Typischerweise bezeichnet Lumineszenz Photonen im Bereich von UV- bis IR-Strahlung.

"Biologisches Molekül" oder "Biomolekül" bezieht sich auf jede Art von Molekülen, die in biologischen Anwendungen vorkommen. Nicht-einschränkend seien aufgezählt: Optional glycosylierte Proteine, z. B. Antikörper, Nanokörper (in der angelsächsischen Literatur: "nano bodies"), Teile von Antikörpern, wie Einzelketten-Antikörper, Fab-Fragmente, virale Proteine, Lektine, Peptide, einschließlich Polyaminosäuren, Nukleinsäuren, einschließlich Desoxyribonukleinsäuren (DNS, DNA), Ribonukleinsäuren (RNS, RNA, siRNA, miRNA) "locked nucleic acid" (LNA), Aptamere, Lipide. Steroide, Botenstoffe, Prione, Kohlenhydrate, kleine Moleküle, usw.

Der Begriff "Mizelle" bezeichnet allgemein eine kolloide Anordnung von Tensiden, die in einer Flüssigkeit verteilt ist und der Begriff "Tensid" bezeichnet eine oberflächenaktive Substanz, die die Oberflächenspannung einer Flüssigkeit herabsetzt, üblicherweise organische Verbindungen, die amphiphil sind. Der Begriff "Transfektion" bezeichnet das beabsichtigte oder unbeabsichtigte Einbringen von Material, so z. B. mizellar verkapselte Nanopartikel in Zellen. Der Begriff "Markieren" oder "Markierung" bezeichnet allgemein das beabsichtigte oder unbeabsichtigte Anheften von Material, so z. B. mizellar verkapselte Nanopartikel an eine Zelle oder ein biologisches Molekül, oder eine biologische Probe. Besagtes Material kann dabei z. B. auf oder in der Zelle angeheftet werden. Transfektion und Markieren einer Zelle oder das Markieren eines biologischen Moleküls, bzw. einer biologischen Probe sind jeweils Beispiele für eine biologische Anwendung. Der Begriff "biologisches Ereignis" schließt eine Wechselwirkung biologischer Strukturen, einen biologischen Prozess, strukturelle Veränderungen in einer biologischen Verbindung oder eine Veränderung in einem biologischen Prozess, ein. Unter dem Begriff "biologische Probe" wird eine Gewebsprobe oder Flüssigkeit verstanden, die aus einem Individuum stammt, einschließlich aber nicht eingeschränkt auf z. B. Plasma, Serum, Spinalflüssigkeit, Sperma, Lymphflüssigkeit, äußere Abschnitte der Haut, des Atmungsapparats, des Verdauungsapparats, des Urogenitaltrakts, Tränenflüssigkeit, Speichel, Milch, Blutzellen, Tumoren, Organe, sowie Proben aus in vitro Zellkultur-Bestandteilen, (einschließlich aber nicht eingeschränkt auf aufbereitetes Zellkulturmedium das aus Zellwachstum in Zellkulturen stammt, wahlweise aus viral infizierten Zellen, rekombinanten Zellen, und Zellbestandteilen) Begriffe wie "biofunktionalisiert", "verbunden", "angeheftet", verknüpft" oder "konjugiert", werden hier gegeneinander austauschbar verwendet und umfassen sowohl direkte als auch indirekte Verknüpfung, Anheftung oder Konjugation, sofern der Zusammenhang nichts anderes verlangt.

Der Begriff "zwitterionisches Nanopartikel" bedeutet hier ein räumliches Objekt, das eine Ausdehnung von 1 bis 1000 nm in wenigstens einer Raumrichtung hat und besagte Ausdehnung von einer zwitterionischen Hülle umschlossen wird. In verschiedenen Ausführungsformen kann diese Hülle aus nicht kovalent mit einander verbundenen Einzelmolekülen bestehen. Die äußere Form der Hülle kann in verschiedenen Ausführungsformen der Erfindung durch ionische, nicht-ionische, elektrostatische, oder kovalente Wechselwirkungen erzeugt werden, auf thermodynamischen, wie z.B. negentropischen Effekten, wie z.B. Mizellenbildung beruhen, oder aus Kombinationen dieser Bindungskräfte. Wahlweise kann die Ausbildung dieser Hülle durch Assoziation der Hüllenkomponenten auf mindestens einem formgebenden anorganischen Nanokristall, wie z.B. einem aus Halbleitern, Silikaten, Salzen, Metallen, oder Metalloxiden bestehender Nanokristall oder auf einem aus organischen Bestandteilen, wie z.B. Polymeren oder festen Lipiden ("solid lipids") bestehenden Nanopartikel oder durch intermolekulare Wechselwirkungen ohne Beteiligung eines formgebenden Nanopartikels erzeugt werden. So stellt die vorliegende Erfindung auch eine Zubereitung bereit, die aus wenigstens einer Mizelle in einer nicht-wässrigen Lösung enthält. Besagte Mizelle kann ein oder mehrere Nanopartikel einkapseln, wobei die Nanopartikel monodisperse Nanopartikel sein können. Besagte Mizelle kann wahlweise anstelle eines oder mehrerer Nanopartikel mindestens einen Wirkstoff enthalten. Besagte Mizelle kann wahlweise leer sein.

Der Begriff "zwitterionische Verbindung" schließt die Begriffe "zwitterionischer Ligand" oder "zwitterionische Gruppe" mit ein. Ebenso sind die Begriffe "Zwitterion" und "Betain" austauschbar.

Besagte zwitterionische Verbindungen haben die allgemeine Formel (I),

A- X- [L¹-Z¹- L²- Z²]ₙ Formel (I)

in der A wahlweise eine Gruppe mit Affinität zur Oberfläche des anorganischen Nanopartikels ist, oder ein mindestens zweiwertiges Atom, vorzugsweise aus der Gruppe C, N, O, S, P oder Si ist, das geeignet ist, eine kovalente Bindung zum Nanopartikel auszubilden. X ist ein optionales Verzweigungselement, dergestalt, dass X zusätzlich zu der Bindung an A mindestens eine weitere Bindung zu L₁ aufweist. X ist wahlweise eine Bindung oder ein mindestens zweiwertiges Atom, vorzugsweise aus der Gruppe C, N, O, S, P oder Si. L¹ und L² sind unabhängig voneinander Linkergruppen; Z¹ schließt eine erste geladene oder ionisierbare Gruppe ein; Z² schließt eine zweite geladene oder ionisierbare Gruppe ein, unter der Maßgabe, dass, falls Z¹ oder Z² Ladungen tragen, diese entgegengesetzt sind. n umfasst die ganzen Zahlen zwischen 1 und der maximalen Valenz von X-1.

A umfasst bzw. weist wahlweise mindestens eine Amino-, Mercapto-, Dithiocarbamat-, Carboxylat-, Phosphat-, Phosphonat-Gruppe auf.

L¹ und L² sind unabhängig voneinander lineare oder verzweigte, wahlweise gesättigte oder ungesättigte Kohlenwasserstoffketten mit ein bis vierzig C-Atomen, die wahlweise Heteroatome aus der Gruppe N, O, P, Si und S enthalten können.

Die folgenden Beispiele dienen zur Veranschaulichung der Erfindung ohne deren Umfang jedoch einzuschränken.

### Referenzbeispiel 1

### Darstellung des tert-Butyl(3-(dimethylamino)propyl)(methyl)carbamats

N,N,N'-Trimethylpropan-1,3-diamin (586 µL, 3,98 mmol) und Triethylamin (TEA) (1,6 mL, 11,54 mmol) wurden in trockenem Dichlormethan (DCM) (12 mL) gelöst und in einem Eisbad abgekühlt. Im Anschluss wurde Di-*tert*-butyldicarbonat (1 mL, 4,35 mmol) in trockenem DCM (2 mL) gelöst und bei einer Temperatur unter 2 °C langsam hinzugetropft. Die Reaktionsmischung wurde für weitere 45 Minuten bei 0 °C und über Nacht (15 Stunden) bei Raumtemperatur gerührt. Zur Aufarbeitung wurde die organische Phase mit gesättigter NaCl, gesättigter NaHCO₃-Lösung und H₂O (je 5 mL) extrahiert, über Na₂SO₄ getrocknet und das Lösungsmittel im Vakuum entfernt. Das Produkt lag in Form eines hellgelben Öls vor.
Ausbeute: 87%

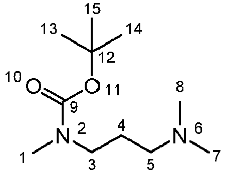

¹H-NMR (600 MHz, CDCl₃): δ [ppm] = 7,26 (CDCl₃); 3,28 - 3,20 (m, 2H, H-3); 2,84 (s, 3H, H-1); 2,31 - 2,25 (m, 2H, H-5); 2,23 (s, 6H, H-7 und H-8); 1,73 - 1,66 (m, 2H, H-4) und 1,45 (s, 9H, H-13, H-14 und H-15).

### Referenzbeispiel 2

### Darstellung des 3-((3-((tert-Butoxycarbonyl)(methyl)amino)propyl)-di-methylammonio)-propan-1-sulfonats

*tert*-Butyl(3-(dimethylamino)propyl)(methyl)carbamat (1,11 g, 5,13 mmol) und 1,3-Propansulton (677 µL, 7,70 mmol) wurden in trockenem DCM (25 mL) gelöst. Die Reaktionsmischung wurde für fünf Tage bei Raumtemperatur gerührt, wobei sich ein perlmuttfarbener Feststoff bildete. Das Lösungsmittel wurde weitestgehend am Rotationsverdampfer entfernt und der Überschuss 1,3-Propansulton durch mehrmaliges Waschen mit trockenem THF abgetrennt. Der Rückstand wurde im Vakuum getrocknet und das Produkt lag als farbloser Feststoff vor.
Ausbeute: 81%

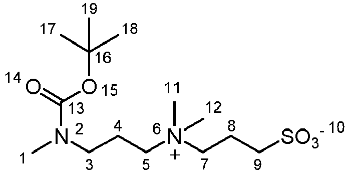

¹H-NMR (400 MHz, D₂O): δ [ppm] = 4,79 (D₂O); 3,55 - 3,51 (m, 2H, H-7); 3,42 - 3,35 (m, 4H, H-3 und H-5); 3,16 (s, 6H, H-11 und H-12); 3,02 (t, 2H, J = 7,2 Hz, H-9), 2,91 (s, 3H, H-1); 2,29-2,21 (m, 2H, H-8), 2,12-2,04 (m, 2H, H-4) und 1,50 (s, 9H, H-17, H-18 und H-19).

¹³C-NMR (100 MHz, D₂O): δ [ppm] = 157,6 (C-13); 81,8 (C-16); 62,3 (C-5); 62.0 (C-7); 50,6 (C-11 und C-12); 47,2 (C-9); 45.0 (C-3); 33,8 (C-1); 27,7 (C-17, C-18 und C-19); 20,8 (C-4) und 18,2 (C-8).

ESI-MS: (m/z) ber. für C₁₄H₃₁N₂O₅S (M + H)⁺ 339, 1948, gef. 339,1955.

IR (neat): u [cm⁻¹] = 2973; 2925; 1679 (C=O); 1199 (S=O); 1161 (C-O); 1034 (S=O), 605 und 524.

### Referenzbeispiel 3

Darstellung des 3-(Dimethyl(3-(methylamino)propyl)ammonio)propan-1-sulfonats 3-((3-((*tert*-Butoxycarbonyl)(methyl)amino)propyl)dimethylammonio)-propan-1-sulfonat (435 mg, 1,29 mmol) wurde in Reinstwasser (13 mL) gelöst und das Reaktionsgefäß mit Stickstoff gespült. Anschließend wurde die Lösung für 90 Minuten in der Mikrowelle auf 135 °C erhitzt. Nach Entfernen des Wassers wurde das Rohprodukt in MeOH (8 mL) gelöst, mit Amberlyst A26 versetzt und vier Stunden bei Raumtemperatur gerührt. Im Anschluss wurde das Amberlyst abfiltriert und das Lösungsmittel am Rotationsverdampfer entfernt. Zur vollständigen Trocknung wurde der Rückstand in wenig Reinstwasser aufgenommen und lyophilisiert. Das Produkt lag als farbloser, stark hygroskopischer Feststoff vor.
Ausbeute: 88%

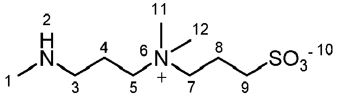

¹H-NMR (400 MHz, D₂O): δ [ppm] = 4,79 (D₂O); 3,52 - 3,48 (m, 2H, H-7); 3,41 - 3,37 (m, 2H, H-5); 3,13 (s, 6 H, H-11 und H-12); 3,00 (t, 2H, J = 7,1 Hz, H-9); 2,76 - 2,72 (m, 2H, H-3); 2,42 (s, 3H, H-1); 2,27 - 2,19 (m, 2H, H-8) und 2,06 - 1,99 (m, 2H, H-4).

¹³C-NMR (100 MHz, D₂O): δ [ppm] = 62,2 (C-7); 61,9 (C-5); 50,7 (C-11 und C-12); 47,1 (C-9); 46,6 (C-3); 34,1 (C-1); 21,2 (C-4) und 18,1 (C-8).

### ESI-MS: (m/z) ber. für C₉H₂₃N₂O₃S (M + H)⁺ 239,1424, gef. 239,1430.

IR (neat): u [cm⁻¹] = 3432; 3034; 2968; 1164 (S=O); 1032 (S=O), 601 und 521.

### Referenzbeispiel 4

### Darstellung des tert-Butyl-bis(3-(dimethylamino)propyl)carbamats

3,3'-Iminobis-N,N-dimethylpropylamin (891 µL, 4,00 mmol) und TEA (1,6 mL, 11,54 mmol) wurden in trockenem DCM (12 mL) gelöst und in einem Eisbad abgekühlt. Im Anschluss wurde Di-*tert*-butyldicarbonat (1 mL, 4,35 mmol) in trockenem DCM (2 mL) gelöst und bei einer Temperatur unter 3 °C langsam hinzugetropft. Die Reaktionsmischung wurde für weitere 90 Minuten bei 1 °C und über Nacht (15 Stunden) bei Raumtemperatur gerührt. Zur Aufarbeitung wurde die organische Phase mit gesättigter NaCl, gesättigter NaHCO₃-Lösung und H₂O (je 5 mL) extrahiert, über Na₂SO₄ getrocknet und das Lösungsmittel im Vakuum entfernt. Das Produkt lag in Form eines hellgelben Öls vor.
Ausbeute: 68%

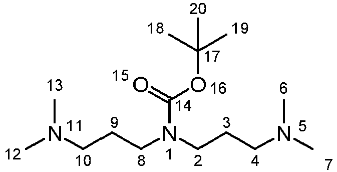

¹H-NMR (400 MHz, CDCl₃): δ [ppm] = 7,26 ppm (CDCl₃); 3,24 - 3,17 (m, 4H, H-2 und H-8); 2,25 (t, 4H, J = 7,4 Hz, H-4 und H-10); 2,21 (s, 12H, H-6, H-7, H-12 und H-13); 1,72 - 1,65 (m, 4H, H-3 und H-9) und 1,45 (s, 9H, H-18, H-19 und H-20).

### Referenzbeispiel 5

### Darstellung des 3,3'-((((tert-Butoxycarbonyl)azadiyl)bis(propan-3,1-diyl))bis(dimethylammoniodiyl))bis(propan-1-sulfonats)

*tert*-Butyl-bis(3-(dimethylamino)propyl)carbamat (500 mg, 1,74 mmol) und 1,3-Propansulton (459 µL, 5,22 mmol) wurden in trockenem DCM (20 mL) gelöst. Nach 24 Stunden hatte sich ein farbloser Feststoff gebildet und die entstandene Suspension war so dickflüssig, dass erneut trockenes DCM (20 mL) hinzugefügt wurde. Die Reaktionsmischung wurde weitere drei Tage bei Raumtemperatur gerührt. Das Lösungsmittel wurde weitestgehend am Rotationsverdampfer entfernt und der Überschuss 1,3-Propansulton durch mehrmaliges Waschen mit trockenem THF abgetrennt. Der Rückstand wurde im Vakuum getrocknet und das Produkt lag als farbloser Feststoff vor.
Ausbeute: 91%

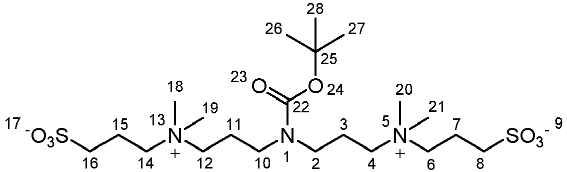

¹H-NMR (400 MHz, D₂O): δ [ppm] = 4,79 (D₂O); 3,55 - 3,51 (m, 4H, H-6 und H-14); 3,40 - 3,36 (m, 8H, H-2, H-4, H-10 und H-12); 3,16 (s, 12H, H-18, H-19, H-20 und H-21); 3,02 (t, 4H, J = 7,1 Hz, H-8 und H-16); 2,27 - 2,21 (m, 4H, H-7 und H-15); 2,13 - 2,05 (m, 4H, H-3 und H-11) und 1,52 (s, 9H, H-26, H-27 und H-28).

¹³C-NMR (100 MHz, D₂O): δ [ppm] = 157,1 (C-22); 82,3 (C-25); 62,2 (C-4, C-6, C-12 und C-14); 50,8 (C-18, C-19, C-20 und C-21); 47,2 (C-8 und C-16); 40,3 (C-2 und C-10); 27,7 (C-26, C-27 und C-28); 22,7 (C-3 und C-11) und 18,2 (C-7 und C-15).

ESI-MS: (m/z) ber. für C₂₁H₄₆N₃O₈S₂ (M + H)⁺ 532,2721, gef. 532,2741.

IR (neat): u [cm⁻¹] = 3452; 3038; 2974; 1676 (C=O), 1157 (C-O); 1033 (S=O), 602 und 521.

### Referenzbeispiel 6

### Darstellung des 3,3'-((Azadiylbis(propan-3,1-diyl))bis(dimethylammoniodiyl))bis-(propan-1-sulfonats)

3,3'-((((*tert*-Butoxycarbonyl)azanediyl)bis(propan-3,1-diyl))bis(dimethylammoniodiyl))bis-(propan-1-sulfonat) (783 mg, 1,47 mmol) wurde in Reinstwasser (14,7 mL) gelöst. Das Reaktionsgefäß wurde mit Stickstoff gespült und die Lösung wurde für 90 Minuten in der Mikrowelle auf 135 °C erhitzt. Nach Lyophilisation wurde das Produkt in MeOH (10 mL) gelöst und über Nacht in Gegenwart von Amberlyst A 26 gerührt. Nach Abfiltrieren des Amberlyst wurde das Lösungsmittel am Rotationsverdampfer entfernt und der Rückstand nach Zugabe von wenig Reinstwasser erneut lyophilisiert. Das Produkt lag in Form eines hellgelben, schaumartigen Feststoffes vor.
Ausbeute: 71%

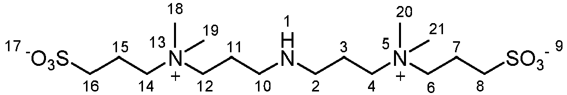

¹H-NMR (400 MHz, D₂O): δ [ppm] = 4,79 (D₂O); 3,55 - 3,51 (m, 4H, H-4 und H-12); 3,44 - 3,39 (4H, m, H-6 und H-14); 3,16 (s, 12 H, H-18, H-19, H-20 und H-21); 3,03 (t, 4H, J = 7,1 Hz, H-8 und H-16); 2,77 - 2,74 (m, 4H, H-2 und H-10); 2,30 - 2,22 (m, 4H, H-7 und H-15) und 2,07 - 1,99 (m, 4H, H-3 und H-11).

¹³C-NMR (100 MHz, D₂O): δ [ppm] = 62,2 (C-4 und C-12); 62,00 (C-6 und C-14); 50,7 (C-18, C-19, C-20 und C-21); 47,2 (C-8 und C-16); 44,9 (C-2 und C-10); 21,7 (C-7 und C-15) und 18,1 (C-3 und C-11).

ESI-MS: (m/z) ber. für C₁₆H₃₈N₃O₆S₂ (M + H)⁺ 432,2197, gef. 432,2216.

IR (neat): u [cm⁻¹] = 3434; 3038; 2968; 2824; 1167 (S=O); 1033 (S=O), 602 und 521.

### Referenzbeispiel 7

### Darstellung des 1-((3-((tert-Butoxycarbonyl)(methyl)amino)dimethyl-ammonio)hex-5-en-3-sulfonats

*tert*-Butyl(3-(dimethylamino)propyl)(methyl)carbamat (434 mg, 2,01 mmol) und 3-Allyl-1,2-oxathiolan-2,2-dioxid (442 mg, 2,72 mmol) wurden in trockenem DCM (20 mL) gelöst. Die Reaktionsmischung wurde für fünf Tage bei Raumtemperatur gerührt, wobei sich ein perlmuttfarbener Feststoff bildete. Das Lösungsmittel wurde weitestgehend am Rotationsverdampfer entfernt und der Überschuss 3-Allyl-1,2-oxathiolan-2,2-dioxid durch mehrmaliges Waschen mit trockenem THF abgetrennt. Der Rückstand wurde im Vakuum getrocknet und das Produkt lag als farbloser Feststoff vor.
Ausbeute: 83%

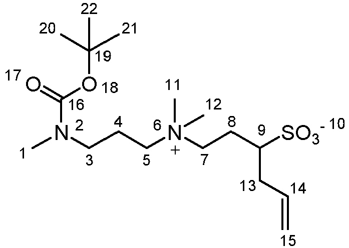

¹H-NMR (400 MHz, D₂O): δ [ppm] = 5,98 - 5,87 (m, 1H, H-14); 5,31 - 5,22 (m, 2H, H-15); 4,79 (D₂O); 3,67 - 3,60 (m, 1H, H-7a); 3,55 - 3,31 (m, 5H, H-3, H-5 und H-7b); 3,14 (s, 6H, H-11 und H-12); 3,04 - 2,98 (m, 1H, H-9), 2,91 (s, 3H, H-1); 2,79 - 2,72 (m, 1H, H-13a); 2,42 - 2,36 (m, 1H, H-13b); 2,26 - 2,14 (m, 2H, H-8), 2,08 - 2,03 (m, 2H, H-4) und 1,50 (s, 9H, H-20, H-21 und H-22).

¹³C-NMR (100 MHz, D₂O): δ [ppm] = 157,1 (C-16); 134,2 (C-14); 118,4 (C-15); 81,8 (C-19); 61,6 (C-5 und C-7); 56,5 (C-9); 50,8 (C-11 und C-12); 45,3 (C-3); 34,0 (C-13); 33,7 (C-1); 27,7 (C-20, C-21 und C-22); 22,2 (C-8) und 20,8 (C-4).

ESI-MS: (m/z) ber. für C₁₇H₃₅N₂O₅S (M + H)⁺ 379,2261, gef. 379,2284.

IR (neat): u [cm⁻¹] = 2976; 1679 (C=O); 1199 (S=O); 1172 (C-O); 1028 (S=O), 602 und 537.

### Referenzbeispiel 8

### Darstellung des 1-(Dimethyl(3-methylamino)propyl)ammonio)hex-5-en-3-sulfonats

1-((3-((*tert*-Butoxycarbonyl)(methyl)amino)dimethylammonio)hex-5-en-3-sulfonat (568 mg, 1,50 mmol) wurde in Reinstwasser (15 mL) gelöst. Das Reaktionsgefäß wurde mit Stickstoff gespült und die Lösung wurde für 90 Minuten in der Mikrowelle auf 135 °C erhitzt. Nach Lyophilisation wurde das Produkt in MeOH (10 mL) gelöst und über Nacht in Gegenwart von Amberlyst A 26 gerührt. Nach Abfiltrieren des Amberlyst wurde das Lösungsmittel am Rotationsverdampfer entfernt und der Rückstand nach Zugabe von wenig Reinstwasser erneut lyophilisiert. Das Produkt lag in Form eines hellgelben, schaumartigen Feststoffes vor.
Ausbeute: 90%

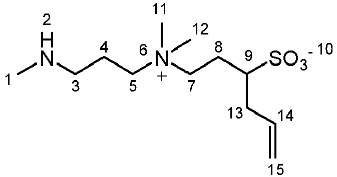

¹H-NMR (400 MHz, D₂O): δ [ppm] = 5,97 - 5,87 (m, 1H, H-14); 5,31 - 5,23 (m, 2H, H-15); 4,79 (D₂O); 3,68 - 3,60 (m, 1H, H-7a); 3,54 - 3,47 (m, 1H, H-7b); 3,41 - 3,37 (m, 2H, H-5); 3,13 (s, 6H, H-11 und H-12); 3,04 - 2,98 (m, 1H, H-9); 2,79 - 2,72 (m, 3H, H-3 und H-13a); 2,43 (s, 3H, H-1); 2,42 - 2,36 (m, 1H, H-13b); 2,27 - 2,11 (m, 2H, H-8) und 2,06 - 1,98 (m, 2H, H-4).

¹³C-NMR (100 MHz, D₂O): δ [ppm] = 134,2 (C-14); 118,4 (C-15); 61, 7 (C-5); 61,5 (C-7); 56,5 (C-9); 50,7 (C-11 und C-12); 46,7 (C-3); 34,2 (C-1); 34,0 (C-13); 22,2 (C-8) und 21,3 (C-4).

ESI-MS: (m/z) ber. für C₁₂H₂₇N₂O₃S (M + H)⁺ 279,1737, gef. 279,1751.

IR (neat): u [cm⁻¹] = 3453; 3028; 2942; 2847; 2793; 1173 (S=O); 1028 (S=O), 601 und 532.

### Referenzbeispiel 9

### Darstellung des 11-Brom-N-methylundecanamids

11-Bromundecansäure (5,00 g, 18,85 mmol) wurde in trockenem DCM (100 mL) gelöst und innerhalb von fünf Minuten wurde eine Lösung aus Oxalylchlorid (1,7 mL, 19,82 mmol) in trockenem DCM (10 mL) zugetropft. Es wurde zu Beginn eine Gasentwicklung beobachtet und die Reaktionsmischung wurde für eine Stunde bei Raumtemperatur gerührt. Im Anschluss wurde das Lösungsmittel und überschüssiges Oxalylchlorid im Vakuum entfernt und das Rohprodukt in trockenem Et₂O (12 mL) gelöst. Die aktivierte Säure wurde zu einer im Eisbad gekühlten wässrigen Methylaminlösung (40%ig, 4,3 mL, 49,80 mmol) getropft, wobei sich sofort ein farbloser Niederschlag bildete. Der Niederschlag wurde abfiltriert und mit H₂O (50 mL) gewaschen. Das Produkt lag nach Trocknung im Vakuum als farbloser Feststoff vor.
Ausbeute: 89%

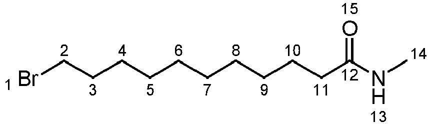

¹H-NMR (400 MHz, CDCl₃): δ [ppm] = 7,26 (CDCl₃); 5,82 (brs, 1H, NH); 3,40 (t, 2H, J = 6,9 Hz, H-2); 2,82 (s, 3H, H-14); 2,21 (t, 2H, J = 7,6 Hz, H-11); 1,88 - 1,81 (m, 2H, H-3); 1,65 - 1,60 (m, 2H, H-10); 1,43 - 1,38 (m, 2H, H-4) und 1,34 - 1,25 (m, 10H, H-5 bis H-9).

### Referenzbeispiel 10

### Darstellung des 11-(Dimethylamino)-N-methylundecanamids

11-Brom-N-methylundecanamid (1,39 g, 5,00 mmol) und eine Lösung aus Dimethylamin in EtOH (33 Gew.%, 9 mL, 50,40 mmol) wurden für zwei Stunden in der Mikrowelle auf 60 °C erhitzt. Das Lösungsmittel und überschüssiges Dimethylamin wurden im Vakuum entfernt und das Hydrobromid des Produkts wurde in Form eines hellgelben Feststoffes erhalten. Der Feststoff wurde in MTBE (10 mL) suspendiert und mit 1 M NaOH (5 mL) extrahiert. Die organische Phase wurde über Na₂SO₄ getrocknet und das Lösungsmittel im Vakuum entfernt. Das Produkt lag in Form eines hellgelben Feststoffes vor.
Ausbeute: 91%

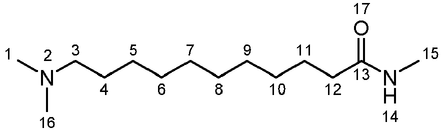

¹H-NMR (400 MHz, CDCl₃): δ [ppm] = 7,26 (CDCl₃); 5,50 (brs, 1H, NH); 2,80 (s, 3H, H-15); 2,33 (t, 2H, J = 7,7 Hz, H-3); 2,29 (s, 6H, H-1 und H-16); 2,15 (t, 2H, J = 7,6 Hz, H-12); 1,65 - 1,57 (m, 2H, H-11); 1,53 - 1,46 (m, 2H, H-4) und 1,34 - 1,22 (m, 12H, H-5 bis H-10).

### Referenzbeispiel 11

### Darstellung des N,N,N'-Trimethylundecan-1,11-diamins

LiAlH₄ (2 M in trockenem THF, 2,7 mL, 5,40 mmol) in trockenem THF (5 mL) wurde in einem Eisbad auf -2 °C abgekühlt. Bei dieser Temperatur wurde eine Lösung von 11-(Dimethylamino)-N-methylundecanamid (660 mg, 2,72 mmol) in trockenem THF (15 mL) langsam zugetropft. Während der Zugabe wurde die Lösung trüb. Nach Zugabe wurde die Reaktionsmischung langsam auf Raumtemperatur erwärmt und im Anschluss unter Rückfluss für drei Stunden erhitzt. Die Reaktionsmischung wurde im Anschluss auf 0 °C abgekühlt und vorsichtig mit H₂O (205 µL), 15%iger NaOH (205 µL) und erneut H₂O (615 µL) gequencht, wobei die anorganischen Salze ausfielen. Die Suspension wurde für weitere 30 Minuten bei Raumtemperatur gerührt. Um den Feststoff abzutrennen, wurde die Suspension zentrifugiert und der Feststoff mehrfach mit trockenem THF gewaschen. Die vereinigten organischen Phasen wurden über Na₂SO₄ getrocknet und das Lösungsmittel wurde im Vakuum entfernt. Das Rohprodukt lag in Form eines gelben Öls vor und wurde ohne weitere Aufarbeitung eingesetzt.
Ausbeute: 96% (Rohausbeute)

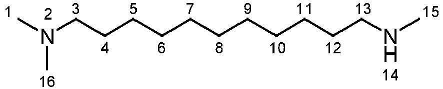

¹H-NMR (600 MHz, CDCl₃): δ [ppm] = 7,26 (CDCl₃); 2,55 (t, 2H, J = 7,2 Hz, H-13); 2,43 (s, 3H, H-15); 2,23 (t, 2H, J = 7,4 Hz, H-3); 2,21 (s, 6H, H-1 und H-16); 1,51 - 1,43 (m, 4H, H-4 und H-12) und 1,34 - 1,23 (m, 14H, H-5 bis H-11).

### Referenzbeispiel 12

### Darstellung des tert-Butyl(11-(dimethylamino)undecyl(methyl)carbamats

Di-*tert*-butyldicarbonat (512 µL, 2,23 mmol) und Amberlyst 15 (76 mg, 15 Gew.%) wurden in einem Rundkolben vorgelegt. Im Anschluss wurde N,N,N'-Trimethylundecan-1,11-diamin (505 mg, 2,21 mmol) hinzugetropft und es wurde eine starke Gasentwicklung beobachtet. Die Reaktionsmischung wurde 15 Minuten gerührt, anschließend mit DCM (22 mL) verdünnt und das Amberlyst abfiltriert. Das Lösungsmittel wurde am Rotationsverdampfer entfernt und das Rohprodukt säulenchromatographisch (DCM/ Methanol/ TEA) gereinigt. Das Produkt lag in Form eines hellgelben Öls vor.
Ausbeute: 57%

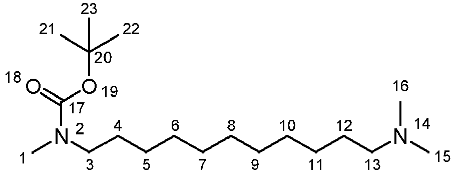

¹H-NMR (400 MHz, CDCl₃): δ [ppm] = 7,26 (CDCl₃); 3,17 (t, 2H, J = 6,9 Hz, H-3); 2,82 (s, 3H, H-1); 2,27 (t, 2H, J = 7,6 Hz, H-13); 2,24 (s, 6H, H-15 und H-16); 1,52 - 1,46 (m, 4H, H-4 und H-12); 1,45 (s, 9H, H-21, H-22 und H-23) und 1,28 - 1.26 (m, 14H, H-5 bis H-11).

¹³C-NMR (100 MHz, CDCl₃): δ [ppm] = 156.0 (C-17); 79,2 (C-20); 77,2 (CDCl₃); 60,0 (C-13); 48,7 (C-3); 45,5 (C-15 und C-16); 34,2 (C-1); 29,7 - 29,5 (C-5 bis C-11); 28,6 (C-21, C-22 und C-23); 27,8 - 27,6 (C-4 und C-12) und 26,9 (C-5 bis C-11).

ESI-MS: (m/z) ber. für C₁₉H₄₁N₂O₂ (M + H)⁺ 329,3163, gef. 329,3169.

IR (neat): u [cm⁻¹] = 2973; 2925; 2854; 1696 (C=O) und 1153 (C-O).

### Referenzbeispiel 13

### Darstellung des 3-((11-((tert-Butoxycarbonyl)(methyl)amino)undecyl)-dimethylammonio)-propan-1-sulfonats

*tert*-Butyl-(11-(dimethylamino)undecyl(methyl)carbamat (250 mg, 0,76 mmol) und 1,3-Propansulton (105 µL, 1,17 mmol) wurden in trockenem DCM (10 mL) gelöst. Die Reaktionsmischung wurde 11 Tage bei Raumtemperatur gerührt. Das Lösungsmittel wurde weitestgehend am Rotationsverdampfer entfernt und der Überschuss 1,3-Propansulton durch mehrmaliges Waschen mit trockenem THF abgetrennt. Der Rückstand wurde im Vakuum getrocknet und das Produkt lag als farbloser Feststoff vor. Ausbeute: 77%

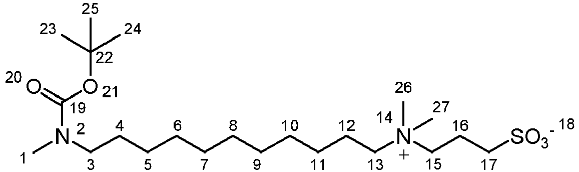

¹H-NMR (600 MHz, D₂O): δ [ppm] = 4,79 (D₂O); 3,50 - 3,47 (m, 2H, H-15); 3,35 - 3,32 (m, 2H, H-13); 3,24 - 3,25 (m, 2H, H-3); 3,12 (s, 6H, H-26 und H-27); 2,99 (t, 2H, J = 7,1 Hz, H-17); 2,84 (s, 3H, H-1); 2,26 - 2,20 (m, 2H, H-16); 1,79 - 1,77 (m, 2H, H-12); 1,57 - 1,53 (m, 2H, H-4); 1,46 (s, 9H, H-23, H-24 und H-25); 1,38 (m, 4H, H-10 und H-11); 1,33 (m, 8H, H-6 bis H-9) und 1,30 - 1,28 (m, 2H, H-5).

¹³C-NMR (150 MHz, D₂O): δ [ppm] = 156,8 (C-19); 81,1 (C-22); 65,1 (C-13); 62,9 (C-15); 51,5 (C-26 und C-27); 49,4 (C-3); 48,2 (C-17); 34,5 (C-1); 29,9 - 29,3 (C-5 bis C-10); 28,8 (C-23, C-24 und C-25); 26,9 (C-4); 26,5 (C-12); 22, 8 (C-11) und 19,0 (C-16).

ESI-MS: (m/z) ber. für C₂₂H₄₆N₂O₅S (M + H)⁺ 451,3200, gef. 451,3214.

IR (neat): u [cm⁻¹] = 3456; 2973; 2925; 2855; 1680 (C=O); 1165 (C-O); 1035 (S=O), 606 und 521.

### Referenzbeispiel 14

### Darstellung des 3-(Dimethyl(11-(methylamino)undecyl)ammonio)propan-1-sulfonats

3-((11-((*tert*-Butoxycarbonyl)(methyl)amino)undecyl)dimethylammonio)-propan-1-sulfonat (600 mg, 1,33 mmol) wurde in Reinstwasser (13 mL) gelöst. Das Reaktionsgefäß wurde mit Stickstoff gespült und die Lösung wurde für 90 Minuten in der Mikrowelle auf 135 °C erhitzt. Das Wasser wurde durch Lyophilisation entfernt und das Produkt lag als farbloser, stark hygroskopischer Feststoff vor.
Ausbeute: 93%

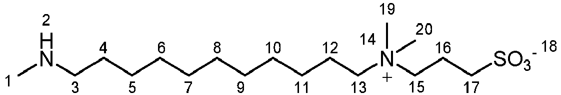

¹H-NMR (400 MHz, D₂O): δ [ppm] = 4,79 (D₂O); 3,51 - 3,46 (m, 2H, H-15); 3,36 - 3,32 (m, 2H, H-13); 3,12 (s, 6H, H-19 und H-20); 3,01 (t, 2H, J = 7,2 Hz, H-17); 2,95 - 2,91 (m, 2H, H-3); 2,63 (s, 3H, H-1); 2,28 - 2,20 (m, 2H, H-16); 1,81 - 1,77 (m, 2H, H-12); 1,69 - 1,61 (m, 2H, H-4); 1,39 (m, 4H, H-5 und H-11) und 1,33 (m, 10H, H-6 bis H-10).

¹³C-NMR (100 MHz, D₂O): δ [ppm] = 64,2 (C-13); 61,9 (C-15); 50,6 (C-19 und C-20); 49,5 (C-3); 47,3 (C-17); 33,0 (C-1); 28,4 - 28,1 (C-6 bis C-10); 26,0 (C-4); 25,7 - 25.4 (C-5 und C-11); 21,7 (C-12) und 18,1 (C-16).

ESI-MS: (m/z) ber. für C₁₇H₃₉N₂O₃S (M + H)⁺ 351,2676, gef. 351,2689.

IR (neat): u [cm⁻¹] = 2919; 2849; 1196 (S=O); 1036 (S=O), 603 und 526.

### Referenzbeispiel 15

### Dithiocarbamat-Bildung

Die Dithiocarbamat-Bildung wurde absorptionsspektroskopisch im Bereich von 230-400 nm verfolgt. Dazu wurden 1 mL einer 25 mM Lösung des Amins gemäß Referenzbeispiel 3 bzw. Referenzbeispiel 14 und 500 µL einer 0,66 M Lösung CS₂ in Me-OH angesetzt. Für die Referenzmessungen der Ausgangssubstanzen wurden je 10 µL der Stammlösungen mit 1.5 mL MeOH in einer UV-Halbmikroküvette verdünnt und die Absorption gemessen. Zur Verfolgung der Dithiocarbamat-Bildung wurden 38 µL der CS₂-Lösung zur restlichen Aminlösung gegeben und die Absorption über einen Zeitraum von 40 bzw. 110 Minuten verfolgt. Dazu wurden 10 µL der Reaktionsmischung nach unterschiedlichen Zeitintervallen mit 1,5 mL MeOH verdünnt und am Absorptionsspektrometer vermessen. Sobald die typischen Absorptionsmaxima bei 255 und 291 nm nicht mehr zunahmen, war die Dithiocarbamat-Bildung abgeschlossen und die Ligandenlösung wurde im nächsten Schritt eingesetzt.

### Referenzbeispiel 16

### Ligandenaustausch

Vor dem Ligandenaustausch wurden die QDQRs (1 nmol) drei Mal mit MeOH gefällt und bei 6.700 x g zentrifugiert. Der farblose Überstand wurde verworfen und die Nanopartikel in 2,5 mL *n*-Hexan aufgenommen. Des Weiteren wurden Verdünnungen der zwitterionischen Amine gemäß Referenzbeispiel 3, Referenzbeispiel 14, Referenzbeispiel 6 und Referenzbeispiel 8 (je 0,2 M) sowie des CS₂ (0,66 M) in MeOH angesetzt. Die Ligandenüberschüsse variierten zwischen 10.000 und 100.000 bezogen auf die Konzentration an Nanopartikeln, wobei das Verhältnis von Amin zu CS₂ 1:1 betrug. Zur Bildung der Dithiocarbamat-Liganden wurden die entsprechenden Mengen Amin und CS₂ auf ein Gesamtvolumen von 1 mL mit MeOH aufgefüllt und 15 Minuten (für die Amine gemäß Referenzbeispiel 3, 14 und 8) bzw. 90 Minuten (für das Amin Referenzbeispiel 6) bei Raumtemperatur gerührt. Im Anschluss wurde die Ligandlösung zu den Nanopartikeln in *n*-Hexan gegeben und das Zweiphasensystem wurde 30 Minuten kräftig gerührt, bis die QDQRs aus der Reaktionslösung ausfielen. In manchen Fällen war eine verlängerte Reaktionszeit von bis zu sechs Stunden notwendig, um eine Fällung der Nanopartikel zu erreichen. Nach kurzer Zentrifugation (1 Minute bei 1.000 x g) wurden die nun farblosen organischen Phasen entfernt, die Nanopartikel im Stickstoffstrom vorsichtig getrocknet und anschließend in Reinstwasser (0,5 - 1 mL) aufgenommen.

### Referenzbeispiel 17

### Stabilitätsmessungen

Für die Stabilitätsmessungen wurden folgende pH-Puffer angesetzt:
Citrat-Puffer pH 5,1
   Citronensäure (960 mg, 5 mmol) wurde in einem 50 mL Maßkolben abgewogen, in Reinstwasser (ca. 40 mL) gelöst und mit Hilfe einer 1 M NaOH auf einen pH Wert von 5,1 eingestellt. Anschließend wurde bis zur Markierung mit Reinstwasser aufgefüllt (Konzentration: 0,1 M).
Phosphat-Puffer pH 7,4
   PBS-Puffer mit einem pH Wert von 7,4 wurde kommerziell erworben.
Borat-Puffer pH 9,0
   H₃BO₃ (62 mg, 1 mmol) wurde in einem 50 mL Maßkolben abgewogen, in Reinstwasser (ca. 40 mL) gelöst und mit Hilfe einer 1 M NaOH auf einen pH Wert von 9,0 eingestellt. Anschließend wurde bis zur Markierung mit Reinstwasser aufgefüllt (Konzentration: 20 mM).
Phosphat-Puffer pH 11,5
   K₂HPO₄ (8,71 g, 50 mmol) wurde in einem 50 mL Maßkolben abgewogen und in wenig Reinstwasser gelöst. Anschließend wurde bis zur Markierung mit Reinstwasser aufgefüllt (Konzentration: 1 M). Von dieser Lösung wurden 500 µL mit 49,5 mL Reinstwasser verdünnt (Konzentration 0,01 M) und mit Hilfe einer 1 M NaOH wurde ein pH Wert von 11,5 eingestellt.

Des Weiteren wurden für die Stabilitätsmessungen folgende Medien verwendet: 10 mM HEPES, DMEM (ohne Phenolrot), 10% FCS in DMEM, PBS und 1% BSA in PBS.

Für die Stabilitätsmessungen wurden 990 µL des pH-Puffers oder Mediums in einer Quarzküvette vorgelegt und 10 µL der Nanopartikellösung hinzugefügt (Endkonzentration: 20 nM). Nach einer Inkubationszeit von zwei Minuten wurde die Fluoreszenzintensität der Proben innerhalb der ersten zwei Stunden alle 20 Minuten, im weiteren Verlauf des ersten Tages stündlich und im Anschluss daran alle 24 Stunden gemessen. Insgesamt wurde die Fluoreszenzintensität über einen Zeitraum von einer Woche verfolgt. Die Messungen wurden bei einer Anregungswellenlänge von 350 nm durchgeführt.

### Beispiel 18

### Zellkulturexperimente

Das Kultivierungsmedium bestand aus 10% FCS in DMEM mit je 1% PenStrep und Natriumpyruvat. Die Inkubation der Zellen fand stets bei 37 °C und einem CO₂-Gehalt von 5% statt.

Die zwitterionischen QuantumDots/QuantumRods(QDQRs) Nanopartikel wurden in diversen *in vitro* Versuchen hinsichtlich ihrer Toxizität und der unspezifischen Zellaufnahme untersucht. Für die Versuche wurden gelb-emittierende QDQRs mit dem kurz-und langkettigen zwitterionischen Liganden (QDQRs-**17a** und QDQRs-**17b**) verwendet, um einen möglichen Einfluss der Kettenlänge des Liganden auf die Wechselwirkungen der QDQRs mit Zellen zu untersuchen. Sowohl für die Toxizitätsuntersuchungen als auch für die Zellaufnahmestudien wurden A549 Zellen verwendet. A549 Zellen sind humane, aus einem Adenokarzinom der Lunge stammende basale Epithelzellen, die als Zelllinie kultiviert wurden.

Für den Einsatz von Nanopartikeln in der Biochemie oder Medizin ist es unabdingbar, dass von ihnen keine intolerable Toxizität ausgeht. Generell besteht bei cadmiumhaltigen Nanopartikeln die Möglichkeit, dass die Zellen durch austretende Cadmiumionen geschädigt werden. Die Wahrscheinlichkeit, dass Cadmiumionen in die Umgebung freigesetzt werden, ist gerade bei den hier verwendeten QDQRs relativ hoch. Zum einen ist der Anteil an Cadmium in den elongierten Nanopartikeln im Vergleich zu sphärischen Partikeln gleichen Durchmessers um den Faktor 12 höher. Zum anderen fehlt den QDQRs eine passivierende Schale aus Zinksulfid, mit der cadmiumhaltige Nanopartikel oftmals umhüllt werden, um das Herauslösen von Cadmiumionen zu verhindern.

Die Toxizität der zwitterionischen QDQRs-**17a** und QDQRs-**17b** wurde mit Hilfe des Cellomics Array Scan geprüft. Für die Toxizitätsuntersuchungen wurden A549-Zellen auf einer 96-Well Platte ausplattiert und mit QDQR-Lösungen in einem Konzentrationsbereich von 25 bis 500 nM inkubiert. Als Positivkontrolle wurde Cadmiumchlorid verwendet, welches in einem Konzentrationsbereich von 25 bis 1.000 µM eingesetzt wurde. Die um den Faktor 1.000 höhere Konzentration an Cadmiumchlorid ist der Tatsache geschuldet, dass in einem einzelnen QDQR der untersuchten Proben etwa 8.200 Cadmiumatome enthalten sind. Unter der Annahme, dass sich die gesamten Nanopartikel auflösen und das in ihnen enthaltene Cadmium freisetzen, entspräche die kleinste eingesetzte Konzentration von 25 nM QDQRs einer Konzentration von 205 µM Cadmiumchlorid. Nach einer Inkubationszeit von 16 Stunden wurden die Zellen mit einer Färbelösung, bestehend aus dem Zellkernfarbstoff Hoechst 33342 und dem Mitochondrienfarbstoff MitoTracker^{®} Deep Red FM, versetzt und danach mit Formaldehydlösung fixiert. Im Anschluss wurden die 96-Well Platten mit dem Cellomics Array Scan ausgelesen. Dabei wurden anhand der Intensität des Hoechstfarbstoffs die Parameter Zellzahl, Zellkernintensität sowie Zellkerngröße und anhand des Mitochondrienfarbstoffs das Transmembranpotential der Mitochondrien mit Hilfe einer Software automatisch bestimmt. Des Weiteren wurden mikroskopische Bilder der Zellen zur Kontrolle der Zellzahl und Zellmorphologie aufgenommen.

### Beispiel 19

### Toxizitätstests

Für die Toxizitätstests wurden A549-Zellen in Kultivierungsmedium auf einer 96-Well Platte ausplattiert (10.000 Zellen pro Well) und über Nacht inkubiert. Nach Absaugen des Mediums wurden die zu testenden Proben (verdünnt mit Kultivierungsmedium, 100 µL pro Well) in unterschiedlichen Konzentrationen aufgetragen und für 16 Stunden inkubiert. Im Anschluss wurden die Proben entfernt und die Zellen mit 100 µL Färbelösung (Endkonzentration: 75 nM MitoTracker^{®} Deep Red und 2,5 µg/ml Hoechst 33342 in DMEM mit 10% FCS) versetzt und weitere 30 Minuten inkubiert. Zur Fixierung der Zellen wurde die Färbelösung entfernt und 100 µL einer warmen Fixierlösung (3,7% Formaldehyd in Dulbecco's PBS, DPBS) hinzugegeben. Nach 20 Minuten wurde die Fixierlösung abgesaugt und zwei Mal mit DPBS gewaschen, bevor die Proben am Cellomics Array Scan vermessen wurden.

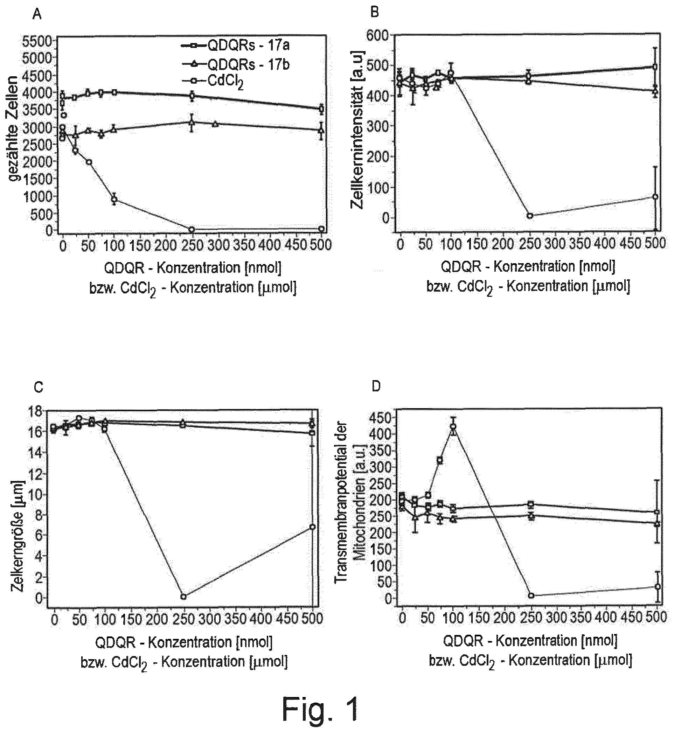

Figur 1 zeigt eine Quantifizierung der Toxizitätstests der QDQRs-17a und QDQRs-17b sowie Cadmiumchlorid (mit (A) Anzahl der Zellen, (B) Intensität des Zellkernfarbstoffs Hoechst 33342, (C) Größe der Zellkerne und (D) Transmembranpotential der Mitochondrien; Anmerkung: Da Cadmiumchlorid bereits ab einer Konzentration von 100 µM eine toxische Wirkung auf die Zellen zeigt, sind in der Darstellung nur die Werte bis 500 µM abgebildet).

In Figur 1 sind die oben genannten Parameter in Abhängigkeit der eingesetzten Konzentration an QDQRs bzw. Cadmiumchlorid gezeigt. Jeder Datenpunkt stellt dabei den Mittelwert aus drei Messwerten dar. Anhand der Diagramme A bis D in Figur 1 wird deutlich, dass sowohl QDQRs-**17a** als auch QDQRs-**17b** einschließlich der höchsten getesteten Konzentration von 500 nM keine erkennbare toxische Wirkung auf die Zellen haben. Die Anzahl der Zellen pro Well (A), die Zellkernintensität (B), die Zellkerngröße (C) und auch das Transmembranpotential der Mitochondrien (D) bleiben über den gesamten Konzentrationsbereich nahezu konstant.

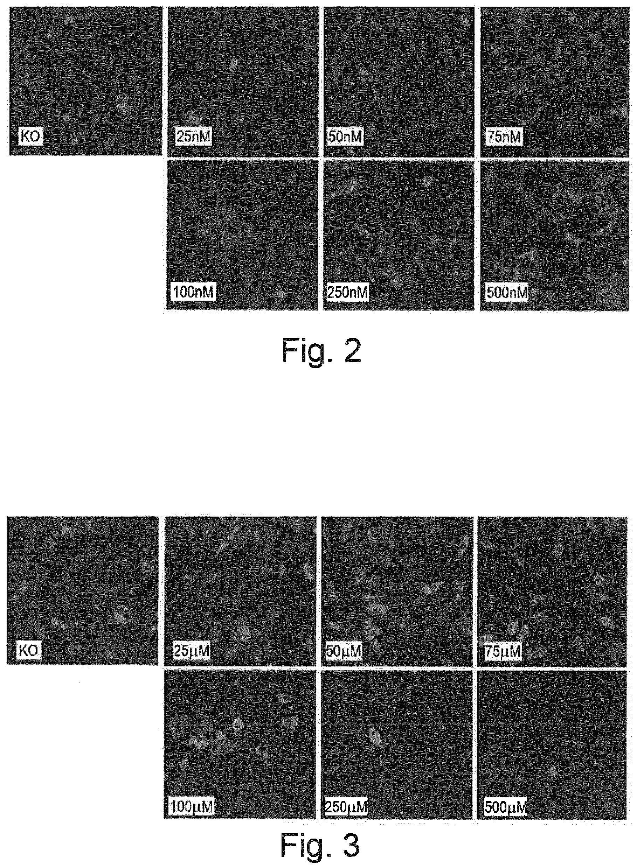

Ebenso sind bei der Betrachtung der mikroskopischen Aufnahmen keine morphologischen Änderungen der Zellen oder Zellkerne nach 16-stündiger Inkubation mit den zwitterionischen QDQRs erkennbar. Als Beispiel hierfür sind in Figur 2 die mikroskopischen Aufnahmen der A549-Zellen nach Inkubation mit QDQRs-**17a** dargestellt. Die Eigenfluoreszenz der QDQRs ist in den Bildern nicht zu sehen, da sie durch entsprechende Filtereinstellungen am Gerät unterdrückt wurde.

Figur 2 zeigt Mikroskopische Aufnahmen der A549-Zellen nach 16-stündiger Inkubationszeit mit unterschiedlichen Konzentrationen von QDQRs-17a. Die Zellkerne sind mit Hoechst 33342 (blau) und die Mitochondrien mit MitoTracker^{®} Deep Red (rot) angefärbt. Zur Kontrolle (KO) ist eine Aufnahme von A549-Zellen nach 16 Stunden gezeigt, die ohne QDQRs inkubiert wurden.

Im Gegensatz dazu ist bei Cadmiumchlorid ab einer Konzentration von 100 µM, die etwa einer QDQR-Konzentration von 12 nM entspricht, eine toxische Wirkung auf die Zellen erkennbar. Die Anzahl an Zellen pro Well, die Zellkernintensität und die Zellkerngröße nimmt signifikant ab, was durch das Absterben der Zellen bedingt ist (Figur 1 A - C). Das Transmembranpotential steigt im Konzentrationsbereich von 50 bis 100 µM erst an und fällt dann wieder ab (Figur 1 D). Dieser Verlauf ist typisch für die toxische Wirkung einer Substanz. Durch Zellstress werden Apoptose-auslösende Faktoren freigesetzt und es kommt zunächst zu einer Zunahme der Mitochondrienmasse, was in der Messung zu einer Intensitätssteigerung des Mitochondrienfarbstoffs führt.

Bei weiterer Erhöhung der Cadmiumchlorid-Konzentration sterben die Zellen zunehmend ab, wodurch die Mitochondrienmasse ebenfalls abnimmt und das Signal des Farbstoffs wieder schwächer wird. Die erneute Zunahme der Zellkernintensität und der Zellkerngröße bei einer Konzentration von 500 µM Cadmiumchlorid sowie die großen Fehlerbalken dieser Messwerte sind dadurch zu erklären, dass bei hoher Toxizität einer Substanz nur noch sehr wenige Zellen im Well vorhanden sind, wodurch weniger Messpunkte für die Auswertung zur Verfügung stehen und die statistischen Fehler entsprechend groß werden.

Besonders deutlich wird dies bei der Betrachtung der mikroskopischen Aufnahmen der Zellen nach 16-stündiger Inkubation mit Cadmiumchlorid, die in Figur 3 dargestellt sind. Ab einer Konzentration von 100 µM Cadmiumchlorid sinkt die Anzahl an Zellen merklich und sie kugeln sich ab. Bei höheren Konzentrationen Cadmiumchlorid sind in den Wells nur noch vereinzelt Zellen zu finden.

Figur 3 zeigt mikroskopische Aufnahmen der A549-Zellen nach 16-stündiger Inkubationszeit mit unterschiedlichen Konzentrationen Cadmiumchlorid. Die Zellkerne sind mit Hoechst 33342 (blau) und die Mitochondrien mit MitoTracker^{®} Deep Red (rot) angefärbt. Zur Kontrolle (KO) ist eine Aufnahme von A549-Zellen nach 16 Stunden gezeigt, die ohne Cadmiumchlorid inkubiert wurden.

Zusammenfassend lässt sich sagen, dass trotz des hohen Cadmiumgehalts in den verwendeten QDQRs und dem Fehlen einer zusätzlich passivierenden Zinksulfidhülle keine messbare Toxizität von den QDQRs bis zu einer Konzentration von 500 nM ausgeht. Damit erfüllen die hergestellten zwitterionischen QDQRs eine wichtige Voraussetzung für spätere biomedizinische Anwendungen.

So konnte gezeigt werden, dass die zwitterionischen QDQRs auch in hohen Konzentrationen von bis zu 500 nM keine toxische Wirkung auf A549-Zellen haben.

Anschließend wurde das Aufnahmeverhalten der QDQRs-**17a** und QDQRs-**17b** mit A549-Zellen analysiert und überprüft, ob die Nanopartikel in Abhängigkeit der Kettenlänge des Liganden unterschiedlich aufgenommen werden.

### Beispiel 20

### Zellaufnahmeversuche mit A549-Zellen (in-vitro)

Für die Zellaufnahmeversuche wurden A549-Zellen in Kultivierungsmedium auf einem 8-Well Labtek ausplattiert (20.000 Zellen pro Well) und 24 bzw. 48 Stunden inkubiert. Das Medium wurde abgesaugt und die zu untersuchenden Proben (verdünnt mit DMEM und entsprechend mit oder ohne 10% FCS, Konzentration: 100 nM) hinzugegeben. Im Anschluss wurden die Zellen für vier bzw. 16 Stunden inkubiert. Nach Ablauf der Inkubationszeit wurde das Medium entfernt, die Zellen mit einer Färbelösung (Endkonzentration: 2,5 µg/ml Hoechst 33342 in DMEM, 200 µL pro Well) versetzt und erneut für 30 Minuten inkubiert. Die Fixierung der Zellen erfolgte ebenfalls mit einer 3,7%igen Formaldehyd-Lösung in DPBS für 20 Minuten. Nach zweimaligem Waschen der Zellen mit DPBS wurden die Proben am Konfokalmikroskop vermessen.

Es wurden zum einen zwei unterschiedlich lange Inkubationszeiten (vier und 16 Stunden) getestet. Zum anderen wurde die Inkubation entweder in Gegenwart oder Abwesenheit von 10% FCS durchgeführt. Durch die Abwesenheit von FCS im Medium sollte untersucht werden, ob die Nanopartikelaufnahme gegebenenfalls in die hungernden Zellen forciert werden kann. In allen Experimenten wurden die Zellen mit einer 100 nM QDQR-Lösung inkubiert. Nach der Inkubation wurden die Zellen gewaschen, die Zellkerne mit Hoechst 33342 gefärbt, fixiert und mit Hilfe eines Konfokalmikroskops analysiert.

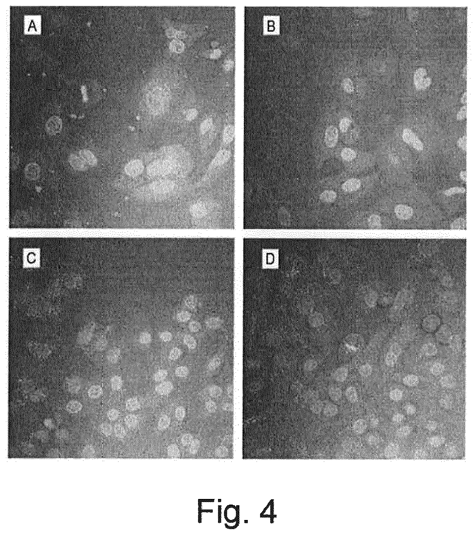

Für QDQRs-**17a** war unter allen experimentellen Bedingungen eine unspezifische Aufnahme der Nanopartikel zu erkennen, siehe Figur 4. Die Aufnahme nach vier Stunden (A und B) war dabei nicht so stark ausgeprägt wie nach 16 Stunden (C und D). Nach vierstündiger Inkubation befanden sich die QDQRs zudem vornehmlich in der Nähe der Zellmembran, wohingegen die Nanopartikel nach längerer Inkubationszeit mehr oder weniger gleichmäßig im Zellinneren verteilt waren. Die Fluoreszenz der Nanopartikel war dabei oftmals in punktförmigen Strukturen zu erkennen, bei denen es sich mutmaßlich um mit QDQRs gefüllte Endosomen handelt. Da A549-Zellen keine phagozytierenden Zellen sind, gelangen die QDQRs höchstwahrscheinlich über unspezifische Endozytose in die Zellen. Außerdem wurden die QDQRs nach vier Stunden Inkubationszeit in Gegenwart von 10% FCS (B) im Vergleich zum Experiment unter serumfreien Bedingung (A) deutlich weniger von den Zellen aufgenommen. Eine reduzierte Aufnahme von Nanopartikeln in serumhaltigem Medium ist bereits aus der Literatur bekannt, wobei die verringerte Aufnahme auf das Vorhandensein einer Proteinkorona zurückgeführt wird. Obwohl zwitterionische Liganden auf Nanopartikeln die Bildung einer Proteinkorona verhindern sollen, scheinen die zwitterionischen QDQRs-**17a** mit dem Protein in Lösung zu wechselwirken, was in einer verringerten Zellaufnahme resultiert.

Figur 4 zeigt Konfokalmikroskopische Aufnahmen von A549-Zellen nach der Inkubation mit 100 nM QDQRs-17a für vier Stunden (A und B) und 16 Stunden (C und D). Die Inkubation erfolgte unter serumfreien Bedingungen (A und C) oder in Gegenwart von 10% FCS (B und D). Die Zellkerne wurden mit dem Farbstoff Hoechst 33342 angefärbt (blau). Die roten Bereiche resultieren aus der Fluoreszenz der QDQRs.

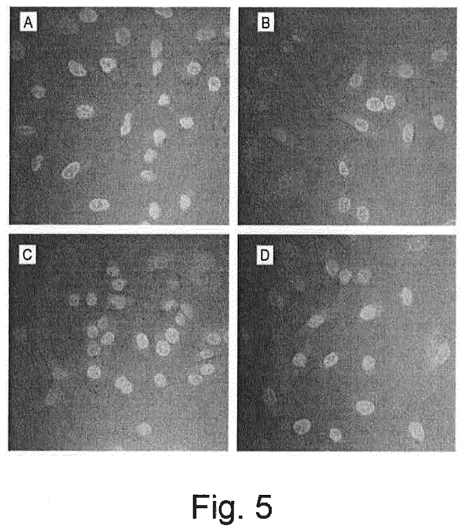

Bei der Inkubation von A549-Zellen mit QDQRs-**17b** zeigte sich überraschenderweise ein völlig anderes Bild (siehe Figur 5).

Figur 5 zeigt konfokalmikroskopische Aufnahmen von A549-Zellen nach der Inkubation mit 100 nM QDQRs-17b für vier Stunden (A und B) und 16 Stunden (C und D). Die Inkubation erfolgte unter serumfreien Bedingungen (A und C) oder in Gegenwart von 10% FCS (B und D). Die Zellkerne wurden mit dem Farbstoff Hoechst 33342 angefärbt (blau). Die roten Bereiche resultieren aus der Fluoreszenz der QDQRs.

Die Nanopartikel wurden nach vierstündiger Inkubationszeit weder unter serumhaltigen (B) noch unter serumfreien Bedingungen (A) von den Zellen aufgenommen. Auch die erhöhte Inkubationszeit von 16 Stunden führte in Gegenwart von 10% FCS zu keiner unspezifischen Aufnahme der QDQRs (D). Lediglich in serumfreiem Medium war eine leichte unspezifische Aufnahme der QDQRs zu erkennen (C), die jedoch im Vergleich zu QDQRs-**17a** deutlich geringer ausfiel.

Allgemein ist Zellaufnahme von Nanopartikeln von vielen verschiedenen Faktoren, wie z. B. der Größe und Form der Partikel, ihrer Oberflächenladung, oder dem Vorhandensein einer Proteinkorona abhängig. Wie anhand der DLS-Messungen und der TEM-Aufnahmen gezeigt werden konnte, sind sowohl die Größe als auch die Form von QDQRs-**17a** und QDQRs-**17b** nahezu identisch, so dass diese Faktoren das unterschiedliche Zellaufnahmeverhalten nicht erklären können.

Zusammenfassend lässt sich sagen, dass sich die QDQRs überraschend deutlich in ihrem Aufnahmeverhalten von A549-Zellen unterscheiden. Somit sind die zwitterionischen Liganden auf der Nanopartikeloberfläche geeignet, die Zellaufnahme durch strukturveränderungen zu beeinflussen.

## Patentansprüche

1. Verwendung eines zwitterionischen Nanopartikels zur unspezifischen Aufnahme in wenigstens eine Zelle, wobei das zwitterionische Nanopartikel wenigstens einen Nanopartikel und eine betainische Hülle aufweist, die den Nanopartikel umschließt, wobei das Nanopartikel ein lumineszenter Halbleiter Nanokristall (SCNC) ist.

2. Verwendung eines zwitterionischen Nanopartikels nach Anspruch 1, **dadurch gekennzeichnet, dass** das Nanopartikel ein magnetisches und/oder plasmonisches Nanopartikel ist.

3. Verwendung eines zwitterionischen Nanopartikels nach Anspruch 1, **dadurch gekennzeichnet, dass** das Nanopartikel wenigstens ein Wirkstoffmolekül enthält.

4. Verwendung eines zwitterionischen Nanopartikels nach Anspruch 1 oder nach Anspruch 2, **dadurch gekennzeichnet, dass** das Nanopartikel ein Halbleiternanopartikel ist, wobei das Nanopartikel mit Schwermetallionen, wie Ag, Cu, Co oder Mn dotiert ist.

5. Verwendung eines zwitterionischen Nanopartikels nach einem der vorhergehenden Ansprüche, **dadurch gekennzeichnet, dass** der Aufbau des Nanopartikels durch die Formel M_{w}NₓA_{y}B_{z} beschrieben wird, wobei M und N unabhängig voneinander ausgewählt sind aus den Elementen der Gruppen 8, 9, 10, 11, 12, 13 oder 14 des Periodensystems der Elemente, z. B., jedoch nicht eingeschränkt auf, Fe, Co, Ni, Pt, Cu, Zn, Cd, Al, Ga, In, Ge, Sn or Pb, und A und B unabhängig voneinander ausgewählt sind aus den Elementen 10, 11, 13, 15 oder 16 des Periodensystems der Elemente, z. B., jedoch nicht eingeschränkt auf Pd, Pt, N, P, As, Sb, Ga, O, S, Se oder Te und wobei w, x, y und z unabhängig voneinander ganzzahlige Vielfache eines Werts zwischen 0 und 1 annehmen kann, unter der Maßgabe, dass die positive Gesamtladung der kationischen Elemente entgegengesetzt gleich der negativen Ladung der anionischen Elemente ist.

6. Verwendung eines zwitterionischen Nanopartikels nach einem der vorhergehenden Ansprüche, **dadurch gekennzeichnet, dass** das Nanopartikel wenigstens von einer Schale umgeben ist.

7. Verwendung eines zwitterionischen Nanopartikels nach einem der vorhergehenden Ansprüchen, **dadurch gekennzeichnet, dass** die zwitterionische Verbindung eine betainische Verbindung ist, die durch die Formel A-X-[L¹-Z¹-L²-Z²]ₙ beschrieben wird, wobei
- A wahlweise eine Gruppe mit Affinität zur Oberfläche des Nanopartikels ist oder ein mindestens zweiwertiges Atom, das geeignet ist, eine kovalente Bindung zum Nanopartikel auszubilden;
- L¹ und L² voneinander unabhängige Linkergruppen sind;
- X wahlweise ein optionales Verzweigungselement dergestalt ist, dass X zusätzlich zu der Bindung an A mindestens eine weitere Bindung zu L¹ aufweist;
- n die ganzen Zahlen zwischen 1 und der maximalen Valenz von X-1 umfasst;
- Z¹ eine erste geladene oder ionisierbare Gruppe einschließt; und
- Z² eine zweite geladene oder ionisierbare Gruppe unter der Maßgabe einschließt, dass, falls Z¹ oder Z² Ladungen tragen, diese entgegengesetzt sind.

8. Verwendung eines zwitterionischen Nanopartikels nach Anspruch 7, **dadurch gekennzeichnet, dass** A aus der Gruppe C, N, O, S, P oder Si gewählt ist, wobei mindestens eine Amino-, Mercapto-, Dithiocabamat-, Carboxy, Phosphat-, Phosphonat-Gruppe vorgesehen ist.

9. Verwendung eines zwitterionischen Nanopartikels nach Anspruch 7, **dadurch gekennzeichnet, dass** L¹ und L² voneinander unabhängige lineare oder verzweigte, wahlweise gesättigte oder ungesättigte Kohlenwasserstoffketten mit ein bis vierzig C-Atomen, die wahlweise Heteroatome aus der Gruppe N, O, P, Si und S enthalten.

10. Verwendung eines zwitterionischen Nanopartikels nach einem der vorhergehenden Ansprüche, **dadurch gekennzeichnet, dass** die zwitterionische Verbindung eine unter
3-(Dimethyl(3-(methylamino)propyl)ammonio)propan-1-sulfonat,
3,3'-((Azadiylbis(propan-3,1-diyl))bis(dimethylammoniodiyl))bis-(propan-1-sulfonat),
1-(Dimethyl(3-methylamino)propyl)ammonio)hex-5-en-3-sulfonat und
3-(Dimethyl(11-(methylamino)undecyl)ammonio)propan-1-sulfonat ausgewählte betainische Verbindung ist.

## Claims

1. Use of a zwitterionic nanoparticle for non-specific incorporation into at least one cell, the zwitterionic nanoparticle comprising at least one nanoparticle and a betaine shell enclosing the nanoparticle, wherein the nanoparticle is a luminescent semiconductor nanocrystal (SCNC).

2. The use of a zwitterionic nanoparticle according to claim 1, **characterized in that** the nanoparticle is a magnetic and/or plasmonic nanoparticle.

3. The use of a zwitterionic nanoparticle according to claim 1, **characterized in that** the nanoparticle comprises at least one active substance molecule.

4. The use of a zwitterionic nanoparticle according to claim 1 or claim 2, **characterized in that** the nanoparticle is a semiconductor nanoparticle, said nanoparticle being doped with heavy metal ions such as Ag, Cu, Co or Mn.

5. The use of a zwitterionic nanoparticle according to any of the preceding claims, **characterized in that** the structure of the nanoparticle is described by the formula M_{w}NₓA_{y}B_{z}, wherein M and N are independently selected from the elements of the groups 8, 9, 10, 11, 12, 13 or 14 of the periodic table of the elements, for instance, but not limited to Fe, Co, Ni, Pt, Cu, Zn, Cd, Al, Ga, In, Ge, Sn or Pb, and A and B are independently selected from the elements 10, 11, 13, 15 or 16 of the periodic table of the elements, for instance, but not limited to Pd, Pt, N, P, As, Sb, Ga, O, S, Se or Te, and wherein w, x, y and z, independently of one another, may be integer multiples of a value between 0 and 1, with the proviso that the total positive charge of the cationic elements is oppositely equal to the negative charge of the anionic elements.

6. The use of a zwitterionic nanoparticle according to any of the preceding claims, **characterized in that** the nanoparticle is surrounded by at least one shell.

7. The use of a zwitterionic nanoparticle according to any of the preceding claims, **characterized in that** the zwitterionic compound is a betaine compound described by the formula A-X-[L¹-Z¹-L²-Z²]ₙ, wherein
- A is selectively a group with affinity to the surface of the nanoparticle or is an at least divalent atom which is suitable to form a covalent bond to the nanoparticle;
- L¹ and L² are linker groups which are independent of each other;
- X is selectively an optional branching element such that X has at least one further bond to L¹ in addition to the bond to A;
- n comprises the integer numbers between 1 and the maximum valence of X-1 ;
- Z¹ includes a first charged or ionizable group; and
- Z² includes a second charged or ionizable group under the proviso that, if Z¹ or Z² carry charges, said charges are opposite.

8. The use of a zwitterionic nanoparticle according to claim 7, **characterized in that** A is selected from the group consisting of C, N, O, S, P or Si, at least one amino, mercapto, dithiocarbamate, carboxyl, phosphate or phosphonate group being provided.

9. The use of a zwitterionic nanoparticle according to claim 7, **characterized in that** L¹ and L² are mutually independent, linear or branched, selectively saturated or unsaturated hydrocarbon chains having one to forty C atoms, which optionally contain heteroatoms from the group consisting of N, O, P, Si and S.

10. The use of a zwitterionic nanoparticle according to any of the preceding claims, **characterized in that** the zwitterionic compound is a betaine compound selected from among
3-(dimethyl(3-(methylamino)propyl)ammonio)propane-1-sulfonate,
3,3'-((azadiylbis(propane-3,1-diyl))bis(dimethylammoniodiyl))bis-(propane-1-sulfonate),
1-(dimethyl(3-methylamino)propyl)ammonio)hex-5-ene-3-sulfonate, and
3-(dimethyl(11-(methylamino)undecyl)ammonio)propane-1-sulfonate.

## Revendications

1. Utilisation d'une nanoparticule zwittérionique pour absorption non spécifique dans au moins une cellule, la nanoparticule zwittérionique comportant au moins une nanoparticule et une enveloppe bétaïnique qui englobe la nanoparticule, la nanoparticule étant un nanocristal semi-conducteur luminescent (SCNC).

2. Utilisation d'une nanoparticule zwittérionique selon la revendication 1, **caractérisée en ce que** la nanoparticule est une nanoparticule magnétique et/ou plasmonique.

3. Utilisation d'une nanoparticule zwittérionique selon la revendication 1, **caractérisée en ce que** la nanoparticule contient au moins une molécule de substance active.

4. Utilisation d'une nanoparticule zwittérionique selon la revendication 1 ou selon la revendication 2, **caractérisée en ce que** la nanoparticule est une nanoparticule de semi-conducteur, la nanoparticule étant dotée d'ions de métaux lourds, tels que Ag, Cu, Co ou Mn.

5. Utilisation d'une nanoparticule zwittérionique selon l'une quelconque des revendications précédentes, **caractérisée en ce que** la structure de la nanoparticule est décrite par la formule M_{w}NₓA_{y}B_{z}, M et N étant choisis indépendamment l'un de l'autre à partir des éléments des groupes 8, 9, 10, 11, 12, 13 ou 14 du système périodique des éléments, par exemple, toutefois sans s'y limiter, Fe, Co, Ni, Pt, Cu, Zn, Cd, Al, Ga, In, Ge, Sn or Pb, et A et B étant choisis indépendamment l'un de l'autre à partir des éléments 10, 11, 13, 15 ou 16 du système périodique des éléments, par exemple, toutefois sans s'y limiter, Pd, Pt, N, P, As, Sb, Ga, O, S, Se ou Te et w, x, y et z pouvant prendre la forme, indépendamment les uns des autres, d'un multiple entier d'une valeur entre 0 et 1, à condition que la charge positive totale des éléments cationique soit égale de façon opposée à la charge négative des éléments anioniques.

6. Utilisation d'une nanoparticule zwittérionique selon l'une quelconque des revendications précédentes, **caractérisée en ce que** la nanoparticule est entourée au moins par une enveloppe.

7. Utilisation d'une nanoparticule zwittérionique selon l'une quelconque des revendications précédentes, **caractérisée en ce que** la liaison zwittérionique est une liaison bétaïnique qui est décrite par la formule A-X-[L¹-Z¹-L²-Z²]ₙ où :
- A est au choix un groupe avec une affinité par rapport à la surface de la nanoparticule ou un atome à au moins deux valeurs qui est adapté pour former une liaison covalente avec la nanoparticule ;
- L¹ et L² sont des groupes de liants indépendants les uns des autres ;
- X est au choix un élément ramifié optionnel tel que X comporte en sus de la liaison à A au moins une autre liaison à L¹ ;
- n comprend les nombres entiers entre 1 et la valence maximale de X-1 ;
- Z¹ inclut un premier groupe chargé ou pouvant être ionisé ; et
- Z² inclut un deuxième groupe chargé ou pouvant être ionisé à condition que si Z¹ ou Z² portent des charges, celles-ci soient opposées.

8. Utilisation d'une nanoparticule zwittérionique selon la revendication 7, **caractérisée en ce que** A est choisi dans le groupe C, N, O, S, P ou Si, au moins un groupe amino, mercapto, dithioca-bamate, carboxy, phosphate, ou phosphonate étant prévu.

9. Utilisation d'une nanoparticule zwittérionique selon la revendication 7, **caractérisée en ce que** L¹ et L² contiennent des chaînes d'hydrocarbure saturées ou insaturées linéaires ou ramifiées indépendantes les unes des autres, au choix avec un à quarante atomes C contenant au choix des hétéroatomes du groupe N, O, P, Si et S.

10. Utilisation d'une nanoparticule zwittérionique selon l'une quelconque des revendications précédentes, **caractérisée en ce que** la liaison zwittérionique est une liaison bétaïnique sélectionnée ci-dessous :
3-(diméthyl(3-(méthylamino)propyl)ammonio)propane-1-sulfonate,
3,3'-((azadiylbis(propane-3,1-diyl))bis(diméthylammoniodiyl))bis-(propane-1-sulfonate),
1-(diméthyl(3-méthylamino)propyl)ammonio)hex-5-en-3-sulfonate et
3-(diméthyl(11-(méthylamino)undécyl)ammonio)propane-1-sulfonate.
